# EUROPEAN PATENT APPLICATION

(11) **EP 4 299 558 A1**
(43) Date of publication of application: **03.01.2024**
(21) Application number: 22305933.8
(22) Date of filing: 27.06.2022
(51) Int. Cl.: C07C 257/18, C07F 3/06, C07F 5/06

(54) **PHENOXY-AMIDINE LIGANDS AND COMPLEXES**

(71) Applicant: Université de Bourgogne, 21000 Dijon (FR); Centre national de la recherche scientifique, 75016 Paris (FR)
(72) Inventor: Le Gendre, Pierre, Norges la Ville (FR); Malacea Kabbara, Raluca, Sennecey Les Dijon (FR); Bayardon, Jérôme, Dijon (FR); Chotard, Florian, Paris (FR); Vaillant-Coindard, Valentin, Tournus (FR); Theron, Benjamin, Saint Apollinaire (FR)
(74) Representative: Bandpay & Greuter

(57) **Abstract**

The invention relates to compounds of formula (I) : 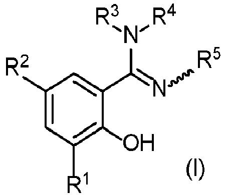 as well as stereoisomeric forms, mixtures of stereoisomeric forms,
and salts thereof, wherein R₁, R₂, R₃, R₄ and R₅ are as defined in claim 1. Compounds of formula (I) are particularly useful for preparing coordination metal or metalloid complexes for the catalysis of ring opening polymerisation of cyclic esters.

## Description

### Technical Field

The present invention relates to a class of ligands related to phenoxy-imines (FI) and bis(phenoxy-imines), also known as Salen in which the imine function is replaced by trisubstituted amidine, giving rise to new phenoxy-amidine and bis(phenoxy-amidine) ligands of formula (I).

The present invention also relates to coordination complexes involving these ligands and application in Ring Opening Polymerisation catalysis of cyclic esters (ROP).

### Background of the invention

Phenoxy-imines and related bridged tetradentate Salen ligands represent one of the most useful classes of ligands for metals. Their ease of access and structural modularity allow the synthesis of a great variety of metal complexes, thus enabling thorough screening processes and identification of highly performant systems. These compounds, also known under the broader name of Schiff base ligands, can coordinate almost all metal ions in the periodic table. Schiff base complexes have found utility in a plethora of fields ranging from catalysis to organic electronics. In catalysis, FI catalysts have proved to be particularly effective in olefin polymerisation/oligomerisation and for the ring-opening polymerisation (ROP) of cyclic esters. In addition, FI complexes have been used to catalyse a wide array of reactions such as cyclopropanation, silylcyanation of aldehydes, hydroamination etc. The performances of Salen ligands are also impressive, notably their ability to control the stereochemical outcome of the reactions. Salen complexes have been widely used to initiate the stereocontrolled ROP of *rac*-lactide. Jacobsen-Katsuki catalysts (chiral Mn(III)Salen complexes) have proved to be among the best systems for the stereoselective epoxidation of non-functionalised alkenes.

However, one drawback of Schiff base ligands is the sensitivity of the core imine moiety(ies) that can undergo various reactions (hydrolysis, reduction, alkylation...); these deleterious processes can plague the overall catalytic behaviour and performance. The electrophilic character of the imine function further reinforced by the coordination to a transition metal is also problematic by avoiding the use of some reagents for catalyst activation or regeneration. For example, the fact that classical alkylating agents generally cannot be used with FI complexes, which complicates the access to dialkyl species and, consequently, to well-defined metal alkyl cation for use in olefin polymerisation. Moreover, despite alternative strategies have been found to get the desired active cationic alkyl metal species, intramolecular alkyl migration from the metal to the imine remains possible during the polymerisation reaction. In the ROP of cyclic esters, metal alkoxides are considered as ideal initiators for the polymerisation process. However, in Schiff base complexes, the preparation of the alkoxide derivatives is not straightforward notably because the imine function of the FI ligand may undergo Meerwein-Ponndorf-Verley (MPV) reduction from the isoproxy ligand.

Therefore, there remains a need for the development of more robust Schiff base ligands and related complexes.

### Summary of the invention

It now has been discovered new compounds related to phenoxy-imine and bis(phenoxy-imine) ligands in which the imine function is replaced by trisubstituted non cyclic amidine, giving rise to new phenoxy-amidine and bis(phenoxy-amidine) ligands. Amidines differ from imines in that the free nitrogen pair of the amidine group NR₂ is involved in resonance, making them much more basic and stable to nucleophiles than imines (pKa = 12 and 6, respectively). The additional NR₂ group in the amidine fragment thus provides steric protection and electronic density to the otherwise exposed electrophilic imine carbon atom. In addition, the strong σ and π-donor nature of the amidine function give additional stability to the metal ion, which is often necessary for robust catalysts with improved performance in processes of great industrial interest. Therefore, unexpectedly, it has been discovered that this makes phenoxy-amidine metal complexes more robust against protic impurities compared to phenoxy-imine analogues.

Another advantage of the invention is that phenoxy-amidines are structurally very close to phenoxy-imines thus conferring high versatility in coordination chemistry.

Thus, in one aspect, the present invention is directed to compounds of formula (I) :

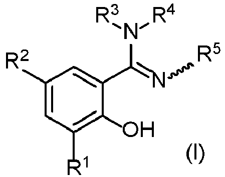

Wherein R¹, R², R³, R⁴ and R⁵ are as defined herein for formula (I),

Another object of the present invention is to provide a coordination complex of formula (III) :

ML'mXn (III)

Wherein :
- M is a metal or a metalloid atom of valence p ;
- X is an halogen atom, C₁-C₆ alkoxy group, N(R²⁷)₂ or C₁-C₆ alkyl;
- R²⁷ is a (C₁-C₆) alkyl group; a SiR¹⁶R¹⁷R¹⁸ group wherein R¹⁶, R¹⁷ and R¹⁸ are as defined in formula (I) ;
- m is 1, 2, 3 or 4 ;
- n is 0, 1, 2, or 3, provided that m+n=p,
- L' is a ligand of formula (I'b), 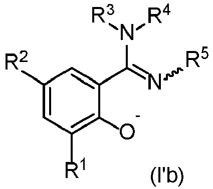
- wherein R¹, R², R³, R⁴ and R⁵ are as defined herein for formula (I'b),

Another object of the present invention relates to the use of a coordination complex of formula (IV) for catalyzing a ring opening polymerisation (ROP) of cyclic esters:

ML"mXn (IV)

Wherein
M, m and n are as defined in any of claims 10 to 12, and
L" is a ligand of formula (I"b), 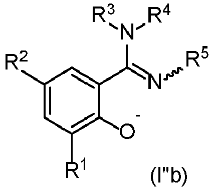 wherein R¹, R², R³, R⁴ and R⁵ are as defined herein for formula (I"b).

A further object of the present invention is to provide a catalysts for use in ring opening polymerisation (ROP) of cyclic esters comprising a coordination complex of formula (III) as defined above.

Advantageously, these catalysts turn out to be particularly robust and able to withstand protic impurities as water and/or lactid acid often present in technical grade monomers like lactide.

These and other objects, features and advantages of the compounds of formula (I) will be disclosed in the following detailed description of the patent disclosure.

### Detailed description

The invention is now described in more detail and in a non-limiting manner in the following description.

### Compounds of formula (I)

In a first aspect, the present invention provides a compound of formula (I) : 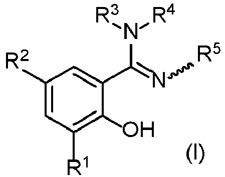 and stereoisomeric forms, mixtures of stereoisomeric forms,
and salts thereof,
Wherein :
R¹ and R² at each occurrence are independently selected from H, F, Cl, Br, I, C₁-C₆ alkyl, C₃-C₁₀ cycloalkyl, C₆-C₁₀ aryl or SiR⁶R⁷R⁸, said alkyl, cycloalkyl and aryl being optionally substituted by 1 to 3 R¹⁹ ;
R³ and R⁴ at each occurrence are independently selected from C₁-C₆ alkyl, C₃-C₁₀ cycloalkyl or C₆-C₁₀ aryl; or
R³ and R⁴ may form together with the nitrogen to which they are attached, a 3-7 membered heterocycloalkyl group,
said alkyl, cycloalkyl, aryl and heterocycloalkyl being optionally substituted by 1 to 3 R²⁰;
R⁵ is C₁-C₆ alkyl, C₁-C₆ perfluoroalkyl, C₃-C₁₀ cycloalkyl, C₆-C₁₀ aryl, C₆-C₁₀ perfluoroaryl, 3-7 membered heterocycloalkyl, 5-7 membered heteroaryl; said alkyl, cycloalkyl, aryl, heteroaryl and heterocycloalkyl being optionally substituted by 1 to 3 R²¹; or
R⁵ is -C(R⁹R¹⁰)-C(R¹¹R¹²)-Y, -Ar-Y, -Ar-Ar-Y or a group of formula (II) : 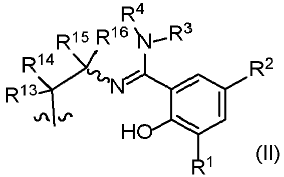
R⁹, R¹⁰, R¹¹and R¹² at each occurrence are independently selected from H, C₁-C₆ alkyl, C₃-C₁₀ cycloalkyl, C₆-C₁₀ aryl, said alkyl, cycloalkyl and aryl being optionally substituted by 1 to 3 R²²; or
R⁹ and R¹¹ represent H and R¹⁰ and R¹² form together with the carbon atoms to which they are attached a C₃-C₁₀ cycloalkyl group, said cycloalkyl being optionally substituted by 1 to 3 R²²; or
Ar is C₆-C₁₀ aryl group optionally substituted by 1 to 3 R²³;
R¹³, R¹⁴, R¹⁵ and R¹⁶ at each occurrence are independently selected from H, C₁-C₆ alkyl, C₃-C₁₀ cycloalkyl or C₆-C₁₀ aryl, said alkyl, cycloalkyl and aryl being optionally substituted by 1 to 3 R²⁴; or
R¹³ and R¹⁵ are H and R¹⁴ and R¹⁶ form together a C₃-C₁₀ cycloalkyl group optionally substituted by 1 to 3 R²⁴ ; or
R¹³, R¹⁴, R¹⁵ and R¹⁶ are combined to form together a C₆-C₁₀ aryl group optionally substituted by 1 to 3 R²⁴ ;
Y represent OH, C₁-C₆ alkoxy, NR¹⁷R¹⁸,
R¹⁷ and R¹⁸ at each occurrence are independently selected from H, C₁-C₆ alkyl, C₃-C₁₀ cycloalkyl or a C₆-C₁₀ aryl, said alkyl, cycloalkyl and aryl being optionally substituted by 1 to 3 R²⁵,
R⁶, R⁷ and R⁸ at each occurrence are independently selected from C₁-C₆ alkyl, or C₆-C₁₀ aryl, said alkyl and aryl being optionally substituted by 1 to 3 R²⁶,
R¹⁹, R²⁰, R²¹, R²², R²³, R²⁴, R²⁵ and R²⁶ at each occurrence are independently selected from C₁-C₄ alkyl, C₁-C₄ alkoxy or C₆-C₁₀ aryl,
With the proviso that
- if R¹ and R² are both H, and R³ and R⁴ are both ethyl, then at least one of R¹³, R¹⁴, R¹⁵ and R¹⁶ is different from H,
- if R¹ and R² are both H, and R³ and R⁴ are both ethyl, then R⁵ cannot be propyl,
- if R¹ and R² are both H, and R³ and R⁴ are both methyl, then R¹³, R¹⁴, R¹⁵ and R¹⁶ cannot form together with the carbon atoms to which they are attached, a cyclohexyl group.

Compounds of formula (I) notably include :
- Proligand compounds which are phenol derivatives, wherein the oxygen is protonated ((I)-OH), and
- Ligand compounds, which are phenolate compounds, wherein the oxygen is deprotonated (I)-O⁻.

Proligand compounds can be directly added to metal precursors in the presence of base (such as NEt₃ or pyridine) or not, or converted before into ligand compounds by reacting the proligand with a base, such as NaH, KHMDS, n-BuLi, sec-BuLi, *tert*-BuLi.

In a preferred embodiment, there are provided compounds of formula (I) having a *trans* configuration stereoisomeric form, of formula (Ia):

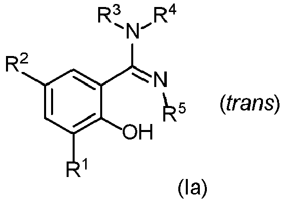

In another preferred embodiment, there are provided compounds of formula (I), wherein R⁵ is a group of formula (II) :

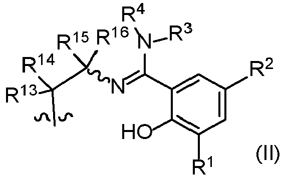

In another preferred embodiment, there are provided compounds of formula (I), wherein which are an alcoholate salt of formula (Ib) :

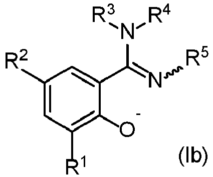

In another preferred embodiment, there are provided compounds of formula (I), which are compounds of formula (Ic) :

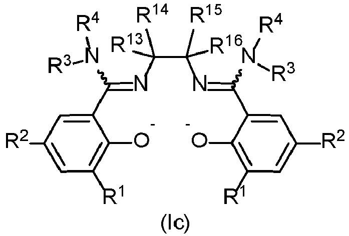

In another preferred embodiment, there are provided compounds of formula (I), wherein R¹ and R² at each occurrence are independently selected from H, or C₁-C₆ alkyl, preferably H, methyl, ethyl, isopropyl, or t-butyl.

In another preferred embodiment, there are provided compounds of formula (I), wherein R³ and R⁴ at each occurrence are independently selected from C₁-C₆ alkyl, or C₃-C₁₀ cycloalkyl, notably methyl, ethyl, isopropyl, cyclohexyl, or R³ and R⁴ may form together with the nitrogen to which they are attached, a pyrrolidinyl or a piperidinyl group.

In another preferred embodiment, there are provided compounds of formula (I), wherein R⁵ is C₁-C₆ alkyl, C₃-C₁₀ cycloalkyl, C₆-C₁₀ aryl, C₆-C₁₀ perfluoroaryl, notably n-propyl, cyclohexyl, or C₆F₅.

In another preferred embodiment, there are provided compounds of formula (I), wherein wherein Y is O⁻, OMe, N(Me)₂ or N(iPr)₂.

### Coordination complexes of formula (III)

Compounds of formula (I) are advantageously able to coordinate a metal or metalloid element thereby forming a coordination complex.

Thus, in a second aspect, the invention relates to a coordination complex of formula (III) :

ML'mXn (III)

Wherein :
- M is a metal or a metalloid element of valence p ;
- X is an halogen atom, C₁-C₆ alkoxy group, N(R²⁷)₂ or C₁-C₆ alkyl;
- R²⁷ is a (C₁-C₆) alkyl group; a SiR¹⁶R¹⁷R¹⁸ group wherein R¹⁶, R¹⁷ and R¹⁸ are as defined in formula (I) ;
- m is 1, 2, 3 or 4 ;
- n is 0, 1, 2, or 3, provided that m+n=p,
- L' is a ligand of formula (I'b), 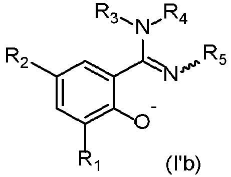
- wherein R¹, R², R³, R⁴ and R⁵ are as defined in formula (I) above, With the proviso that
- if R¹ and R² are both H, and R³ and R⁴ are both methyl, then R¹³, R¹⁴, R¹⁵ and R¹⁶ cannot form together with the carbon atoms to which they are attached, a cyclohexyl group.

In a preferred embodiment, there are provided coordination complexes selected from (IIIa), (IIIb) or (IIIc) :

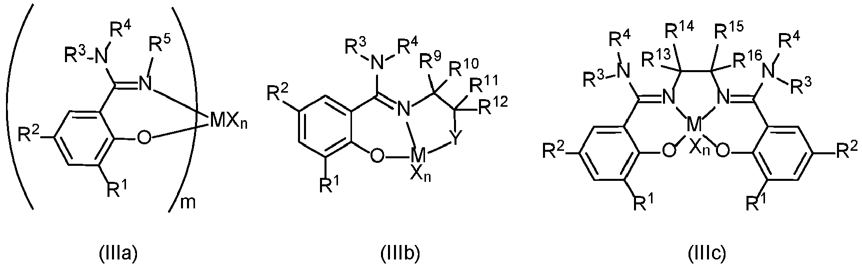

In another preferred embodiment, there are provided coordination complexes of formula (III), wherein M is selected from Ti, Zr, Zn, Al or B.

It is to be noted that the above formula (III) encompasses dimers of complexes of formula (III). Indeed, the coordination complexes of the invention may exist in dimeric forms in the solid state or in non-coordinating solvents, which means that two monomeric complexes, such as two complexes of formula (IIIa), may be bound together by weak bonds so as to form a dimeric complex. An example of such a dimeric complex is given in the examples below :

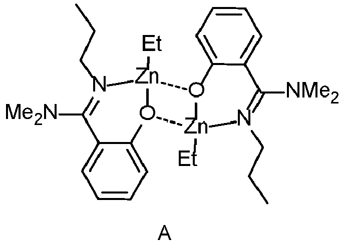

### Use of coordination complexes

The coordination complexes of formula (III) turned out to be particularly useful to catalyze ring opening polymerisation (ROP) of cyclic esters.

As used herein, the term "ring opening polymerisation (ROP)" refers to a reaction in which one polymer chain has a reactive center on its terminal end that reacts with another cyclic monomer, hence opening its ring system to form a longer polymer chain. The reactive center on the terminal end of the polymer chain can be ionic, neutral, or radical. While in the cyclic monomer, the driving force is steric repulsions or bond-angle strain. These cyclic monomers usually contain alkenes, alkanes, or heteroatoms in the ring. The ability of polymerisation and the corresponding driving force varies depending on the type and size of the ring structure. Ring-opening polymerisation has been applied to produce many commercially important polymers including polyesters from cyclic ester (lactones), polysiloxanes from cyclic siloxanes, and polyamides from cyclic amides (lactams).

Thus, in a third aspect, the invention relates to the use of a coordination complex of formula (IV) for catalyzing a ring opening polymerisation (ROP) of cyclic esters :

ML"mXn (IV)

Wherein
M, m and n are as defined in any of claims 10 to 12, and
L" is a ligand of formula (I"b), 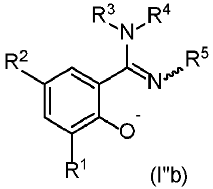 wherein R¹, R², R³, R⁴ and R⁵ are as defined in formula (I) above.

### Catalyst for use in ring opening polymerisation (ROP) of cyclic esters

In a fourth aspect, the invention relates to a catalyst for use in ring opening polymerisation (ROP) of cyclic esters comprising a coordination complex of formula (III), as defined above.

In a preferred aspect, there is provided a catalyst which is the complex of formula (IIIa1) represented as a dimer:

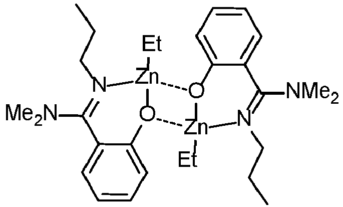

### Definitions

The following terms and expressions contained herein are defined as follows.

As used herein, a range of values in the fonn "x-y" or "x to y", or "x through y", include integers x, y, and the integers therebetween. For example, the phrases "I-6", or "I to 6" or "I through 6" are intended to include the integers 1, 2, 3, 4, 5, and 6. Preferred embodiments include each individual integer in the range, as well as any subcombination of integers. For example, preferred integers for "1-6" can include 1, 2, 3, 4, 5, 6, 1-2, 1-3, 1-4, 1-5, 2-3, 2-4, 2-5, or 2-6, etc.

As used herein, the term "alkyl" refers to a straight-chain, or branched, alkyl group having 1 to 6 carbon atoms, such as methyl, ethyl, propyl, isopropyl, butyl, isobutyl, sec-butyl, tert-butyl, pentyl, isoamyl, neopentyl, 1-ethylpropyl, 3-methylpentyl, 2,2-dimethylbutyl, 2,3-dimethylbutyl, hexyl, etc. The alkyl moiety of alkyl-containing groups, such as alkoxy, alkoxycarbonyl, and alkylamino groups, has the same meaning as alkyl defined above. Lower alkyl groups, which are preferred, are alkyl groups as defined above which contain 1 to 4 carbons, such as methyl, ethyl, propyl, isopropyl, butyl, isobutyl, sec-butyl, and tert-butyl. A designation such as "C₁-C₄ alkyl" refers to an alkyl radical containing from 1 to 4 carbon atoms.

As used herein, the term "cycloalkyl" refers to a saturated or partially saturated mono-, bi- or tricyclic substituted or not substituted alkyl ring system containing 3 to 10 carbon atoms. A designation such as "C₃-C₇ cycloalkyl" refers to a cycloalkyl radical containing from 3 to 7 ring carbon atoms. Preferred cycloalkyl groups include those containing 3, 4, 5, 6, or 7 ring carbon atoms. More preferred cycloalkyl groups include those containing 3, 4, 5, or 6 ring carbon atoms. Examples of cycloalkyl groups include such groups as cyclopropyl, cyclobutyl, cyclopentyl, cyclohexyl, cycloheptyl, cyclooctyl, pinenyl, and adamantanyl.

As used herein, the term "aryl" refers to a substituted or unsubstituted, mono- or bicyclic hydrocarbon aromatic ring system having 6 to 10 ring carbon atoms. Examples include phenyl and naphthyl. Preferred aryl groups include unsubstituted or substituted phenyl and naphthyl groups. Included within the definition of "aryl" are fused ring systems, including, for example, ring systems in which an aromatic ring is fused to a cycloalkyl ring. Examples of such fused ring systems include, for example, indane, indene, and tetrahydronaphthalene.

As used herein, the term "heterocycloalkyl" refers to a 3 to 7 membered cycloalkyl group in which one, two or three ring carbon atoms are replaced by a heteroatom such as -O-, -N-, or -S-. Examples of heterocycloalkyl groups include pyrrolidinyl, pyrrolinyl, imidazolidinyl, imidazolinyl, pirazolidinyl, pirazolinyl, pyrazalinyl, piperidyl, piperazinyl, morpholinyl, thiomorpholinyl, tetrahydrofuranyl, dithiolyl, oxathiolyl, dioxazolyl, oxathiazolyl, pyranyl, oxathiazinyl, and oxadiazinyl.

As used herein, the term "heteroaryl" refers to an aromatic group containing 5 to 14 ring carbon atoms in which one, two three, or four ring carbon atoms are replaced by a heteroatom such as -O-, -N-, -S-, or-Se-. Examples of heteroaryl groups include pyrrolyl, furanyl, thienyl, pyrazolyl, imidazolyl, thiazolyl, isothiazolyl, isoxazolyl, oxazolyl, oxathiolyl, oxadiazolyl, triazolyl, oxatriazolyl, furazanyl, tetrazolyl, pyridyl, pyrazinyl, pyrimidinyl, pyridazinyl, triazinyl, picolinyl, indolyl, isoindolyl, indazolyl, benzofuranyl, isobenzofuranyl, purinyl, quinazolinyl, quinolyl, isoquinolyl, benzoimidazolyl, benzothiazolyl, benzothiophenyl, thianaphthenyl, benzoxazolyl, benzisoxazolyl, cinnolinyl, phthalazinyl, naphthyridinyl, and quinoxalinyl. Included within the definition of "heteroaryl" are fused ring systems, including, for example, ring systems in which an aromatic ring is fused to a heterocycloalkyl ring. Examples of such fused ring systems include, for example, phthalamide, phthalic anhydride, indoline, isoindoline, tetrahydroisoquinoline, chroman, isochroman, chromene, and isochromene.

It is recognized that compounds of the present invention may exist in various stereoisomeric forms, in particular configuration or optical stereoisomers. The compounds of the present invention thus include configuration *cis-trans* stereoisomers depending on the relative position of the substituents with regard to the double bond C=N of the amidine function. Amidines are generally in *trans* form which is thermodynamically more favorable. The proligands will be therefore in *trans* form whereas the ligands in the complexes will be in cis form to allow the nitrogen atom and the oxygen atom of the phenolate group to chelate the metal or metalloid element.

As such, the compounds of the present invention include both diastereomers, enantiomers. The compounds are normally prepared as racemates and can conveniently be used as such, but individual enantiomers can be isolated or synthesized by conventional techniques if so desired. Such racemates and individual enantiomers and mixtures thereof form part of the present invention.

It is well known in the art how to prepare and isolate such optically active forms. Specific stereoisomers can be prepared by stereospecific synthesis using enantiomerically pure or enantiomerically enriched starting materials. The specific stereoisomers of either starting materials or products can be resolved and recovered by techniques known in the art, such as resolution of racemic forms, normal, reverse-phase, and chiral chromatography, recrystallization, enzymatic resolution, or fractional recrystallization of addition salts formed by reagents used for that purpose. Useful methods of resolving and recovering specific stereoisomers described in Eliel, E. L.; ,Wilen, S.H. Stereochemistry of Organic Compounds; Wiley: New York, 1994, and Jacques, J, et al. Enantiomers, Racemates, and Resolutions; Wiley: New York, 1981, each incorporated by reference herein in their entireties.

### Synthesis

The compounds of the present invention may be prepared in a number of methods well known to those skilled in the art, including, but not limited to those described below, or through modifications of these methods by applying standard techniques known to those skilled in the art of organic synthesis. All processes disclosed in association with the present invention are contemplated to be practiced on any scale, including milligram, gram, multigram, kilogram, multikilogram or commercial industrial scale.

It will be appreciated that the compounds of the present invention may contain one or more asymmetrically substituted carbon atoms, and may be isolated in optically active or racemic forms. Thus, all chiral, diastereomeric, racemic forms and all geometric isomeric forms of a structure are intended, unless the specific stereochemistry or isomeric form is specifically indicated. It is well known in the art how to prepare such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from active starting materials or by deliberate chiral synthesis of target centers.

General routes to prepare the Examples of the present invention are shown in the Schemes and examples that follow. The reagents and starting materials are commercially available and/or, using well-known techniques, can be readily synthesized by one of ordinary skill in the art. All substituents in the synthetic Schemes, unless otherwise indicated, are as previously defined.

### Examples

Other features of the invention will become apparent in the course of the following descriptions of exemplary embodiments. These examples are given for illustration of the invention and are not intended to be limiting thereof.

### General procedure for the synthesis of the ligands of formula (I)

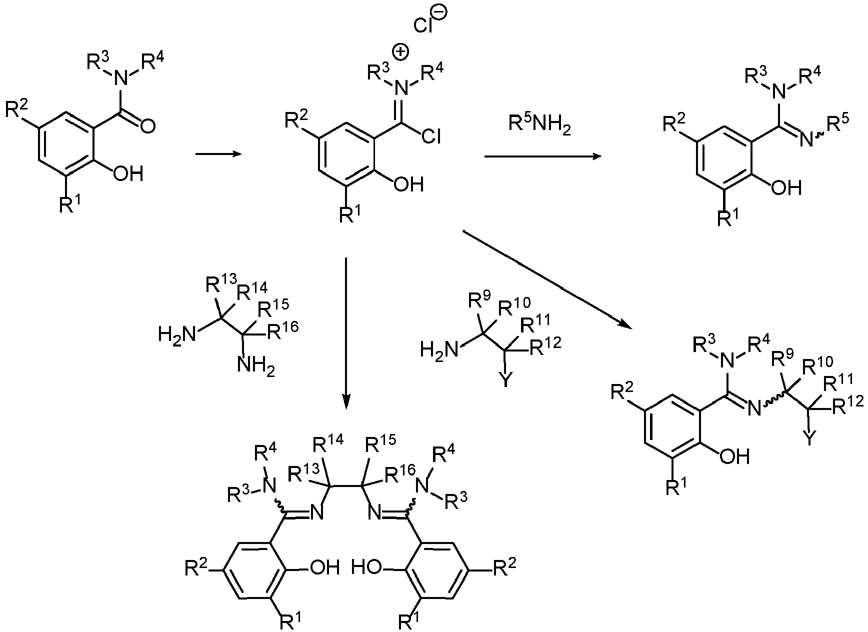

### Materials and Methods

All reactions, except when indicated, were carried out under an atmosphere of argon using conventional Schlenk techniques and Ar glovebox. DCM, diethyl ether, THF, toluene, and pentane were dried using a MBRAUN SPS 800.

Reagents were commercially available and used as received. High resolution mass spectra were recorded on a Thermo LTQ Orbitrap XL ESI-MS (ElectroSpray Ionization Mass Spectrometry). NMR spectra (¹H, ¹³C) were recorded on Bruker 300 Avance Neo, Bruker 400 Avance Neo, Bruker 500 Avance Neo, or Bruker 600 Avance HD spectrometers. All acquisitions were performed at 300 K. Chemical shifts are quoted in parts per million (δ) relative to TMS (for ¹H and ¹³C). For ¹H and ¹³C spectra, values were determined by using solvent residual signals (e.g. CHCl₃ in COCl₃) as internal standards. The apparent multiplicity of the 1H signals is reported. Assignment of ¹H and ¹³C signals (when possible) was done through the use of 2D experiences (COSY, HSQC and HMBC).

### Example 1

### Synthesis of the proligand (Ia) with R¹ = R² = H; R³ = R⁴ = Me; R⁵ = propyl

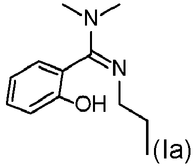

2.0 g of *N*,*N*-dimethyl-2-hydroxybenzamide (12.3 mmol, 1 eq.) were charged in a Schlenk under argon and solubilized in 10 mL of thionyl chloride. The mixture was stirred overnight at r.t. and the solvent was then evaporated under vacuum. 15 mL of dried CH₂Cl₂ were added to solubilize the crude product before being further evaporated. A solution of the crude product and 8.6 mL of triethylamine (61.5 mmol, 5 eq.) in 50 mL of dried CH₂Cl₂ was then cooled to 0°C, and 1 mL of propylamine (12.3 mmol, 1 eq.) was added. The mixture was allowed to reach r.t. and stirred for 2h30. After hydrolysis with 50 mL of distilled water, the product was extracted with 4x50 mL of CH₂Cl₂. The organic phases were combined and dried over MgSO₄ before being evaporated. The crude product was purified by column chromatography on silica first using ethyl acetate then a CH₂Cl₂/MeOH mixture as eluents affording 1.70 g of pure proligand (67% yield).

¹H NMR (400 MHz, CD₂Cl₂, 298 K): δ (ppm) = 11.86 (s, 1H), 7.14 (ddd, *J* = 8.7, 7.1, 1.9 Hz, 1H), 6.83 (dd, J = 7.5, 1.9 Hz, 1H), 6.78 (d, *J* = 11.7 Hz, 1H), 6.52 (t, *J* =7.3 Hz, 1H), 3.01 (overlapping signals, 8H), 1.47 (hex, J = 6.9 Hz, 2H), 0.77 (t, J = 7.4 Hz, 3H).

{¹H}¹³C NMR (100 MHz, CD₂Cl₂, 298 K): δ(ppm) = 165.64, 163.12, 131.81, 128.74, 119.88, 118.43, 113.85, 49.76, 39.99, 24.77, 11.4.

### Example 2

### Synthesis of the proligand (Ic)H₂with R¹ = R² = H; R³ = R⁴ = Me; R¹³, R¹⁴, R¹⁵, and R¹⁶ are combined to form together a phenylene

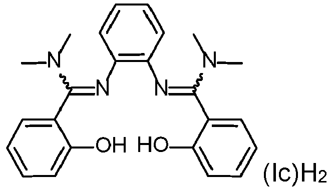

2.0 g of *N*,*N*-dimethyl-2-hydroxybenzamide (12.3 mmol, 1 eq.) were charged in a Schlenk under argon and solubilized in 10 mL of thionyl chloride. The mixture was stirred overnight at r.t. and the solvent was then evaporated under vacuum. 15 mL of dried CH₂Cl₂ were added to solubilize the crude product before being further evaporated. A solution of the crude product and 8.6 mL of triethylamine (61.5 mmol, 5 eq.) in 50 mL of dried CH₂Cl₂ was then cooled to 0°C, and 600 mg of o-phenylenediamine (5.5 mmol, 0.45 eq.) were added. The mixture was allowed to reach r.t. and stirred for 2h30. After hydrolysis with 50 mL of distilled water, the product was extracted with 4x50 mL of CH₂Cl₂. The organic phases were combined and dried over MgSO₄ before being evaporated. The crude product was taken back in 50 mL of diethyl ether and a 1M solution of HCI in diethyl ether was added up to pH=1. The brownish product was filtrated under vacuum and washed with 3x40 mL of diethyl ether. The whitish solid was suspended in 10 mL of distilled water and a 5M aqueous solution of NaOH was added up to pH=9-10. The product was extracted with 50mL of CH₂Cl₂. The organic phase was washed with 3x10 mL of distilled water before being dried over MgSO₄ and evaporated under vacuum. The crude product was purified by column chromatography on silica using MeOH as eluent affording 1.48 g of pure proligand (67% yield).

¹H NMR (600 MHz, CD₂Cl₂, 298 K): δ(ppm) = 7.08 (2H, pseudo td, J =7.7, 1.8 Hz), 6.98 (2H, dd, J = 7.6, 1.8 Hz), 6.80 (2H, broad s), 6.75 (2H, d, *J* = 8.2Hz), 6.57-6.52 (2H, m), 6.40 (2H, broad s), 3.21 (6H, broad m), 2.77 (6H, broad m).

{¹H}¹³C NMR (150 MHz, CD₂Cl₂, 298 K): δ(ppm) = 161.56, 155.30, 144.56, 130.41, 129.08, 124.01, 123.91, 123.37, 120.21, 119.96, 39.86, 37.89.

### Example 3

### Synthesis of the complex (IIIa₁) with R¹ = R² = H; R³ = R⁴ = Me; R⁵ = propyl; M = Zn, X = Et, m = 1, n = 1

In the glovebox, 114.5 mg (0.56 mmol) of (Ia) was solubilized in THF (8 mL) and ZnEt₂ 1M/hexanes (1 eq., 0.56 mmol, 0.56 mL) was added slowly. The mixture was stirred 2 h and then solvent was removed under vacuum. 4 mL of pentane were added and after a few minutes of stirring, the suspension was let to settle and the supernatant was eliminated. The whitish solid was finally dried under vacuum, affording 91 mg of complex IIIa as a white solid (54% yield).

¹H NMR (400.16 MHz, pyridine-Ds, 298 K): δ(ppm) = 7.35 (t, *J* = 7.3 Hz, 1H), 7.19 (d *J* = 6.2 Hz, 1H), 7.11 (d, *J = 8.1* Hz, 1H), 6.77 (t, *J* = 7.2 Hz, 1H), 3.46 (br, 2H), 3.06 (6H, s), 1.76 (m, 2H), 1.67 (t, *J* = 8.1 Hz, 3H), 0.94 (t, *J =* 7.3 Hz, 3H), 0.83 (q, *J* = 8.1 Hz, 2H).

{¹H}¹³C NMR (100.63 MHz, pyridine-Ds, 298K): δ(ppm) = 166.28, 163.81, 130.32, 129.21, 126.42, 120.72, 113.92, 54.04, 38.41, 26.97, 14.69, 13.04, -2.37.

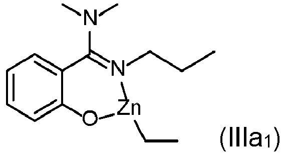

### Example 4

### Synthesis of the complex (IIIa₂) with R¹ = R² = H; R³ = R⁴ = Me; R⁵ = propyl; M = Al, X = Me, m = 1, n = 2

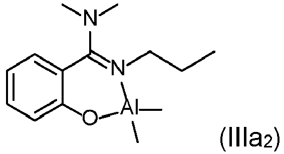

In a glovebox, 61.9 mg (0.3 mmol) of (Ia) were suspended into 3 mL of THF. Then 0.15 mL (0.3 mmol, 1 eq.) of a 2M solution of AIMe₃ in hexane were added and the solution was stirred at r.t. for 2 h. Volatiles were evaporated under vacuum, and the sticky oil obtained was taken back in 4 mL of pentane. The solvent was then evaporated and 3 mL of pentane were added and further evaporated. 3 mL of pentane were used to suspend the white solid obtained. After decantation, the supernatant was eliminated and the white powder dried under vacuum, affording 29 mg (37% yield) of complex (IIIa2) containing about 9% of AIMe₃.

¹H NMR (400 MHz, THF-D₈, 298 K): δ (ppm) = 7.40-7.16 (overlapping signals, 2H), 6.78 (d, *J* = 8.3 Hz, 1H), 6.66 (t, *J* = 7.6 Hz, 1H), 3.50 (br, 2H), 3.01 (s, 6H), 1.67 (m, 2H), 0.99 (t, 3H), -0.98 (6H, s).

{¹H}¹³C NMR (100 MHz, THF-D₈, 298 K): δ (ppm) = 170.08, 166.59, 134.55, 132.48, 123.28, 119.19, 116.79, 50.85, 43.10, 24.45, 11.96, -0.98.

### Example 5

### Synthesis of the complex (IIIc) with R¹ = R² = H; R³= R⁴ = Me; R¹³, R¹⁴, R'⁵ , and R¹⁶ are combined to form together a phenylene: M = Al, X = Me, n = 1

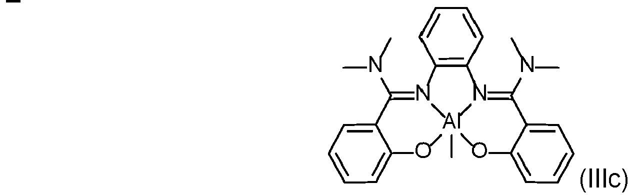

In glovebox, 250µL of AIMe₃ 2M/hexanes (0.5mmol, 1 eq.) were added to a solution of 201.0 mg (0.5mmol, 1 eq.) of (Ic)H₂ in 10mL of dried THF. The mixture was stirred at 60°C for 2h. After cooling down to r.t., the solvents were evaporated under vacuum. The crude product was washed with 8 mL of dried pentane, affording 180 mg of complex (81% yield).

¹H NMR (500 MHz, CD₂Cl₂, 298 K): δ(ppm) = 7.33 (2H, dd, J = 8.0, 1.8 Hz), 7.25 (2H, pseudo ddd, J= 8.7, 7.2, 1.9 Hz), 6.80-6.73 (6H, m), 6.49-6.44 (2H, m), 3.00 (6H, s), 2.90 (6H, s), -1.12 (3H, s).

{¹H}¹³C NMR (125 MHz, CD₂Cl₂, 298 K): δ(ppm) = 165.40, 162.32, 141.99, 133.50, 131.33, 123.41, 122.91, 122.52, 119.71, 117.04, 42.40, 40.57, -13.60.

### Example 6:

### PLA synthesis:

The performance of a phenoxy-amidine ligand complex A for the ring-opening polymerisation (ROP) of lactide was tested. A similar phenoxy-imine complex B was tested in parallel to assess the impact of the amidine moiety on catalytic performance. As shown in Table 1, the two complexes gave comparable results (activity and MW control) when a recrystallized and sublimed lactide was used. On the other hand, it turns out that only the phenoxy-amidine complex A was active in the presence of a lactide contaminated with lactic acid (4 mol%). The ability of this Zn complex with bidentate anionic phenoxy-amidine ligand to withstand lactic acid content as high as 4 mol% (4 equiv/Zn) is unprecedented and is the first proof of concept that phenoxy-amidine ligands can outperform phenoxy-imine ligands in case of stability problems.

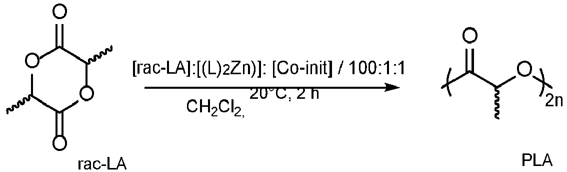

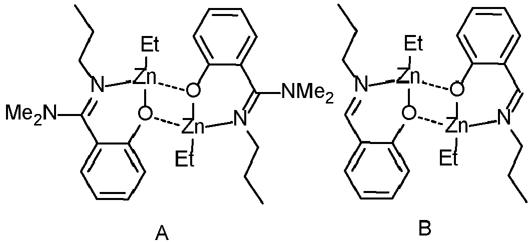

| Init. | Conv (%) | M_{n, exp} (Ð) | M_{n, theo} |
|---|---|---|---|
| A | 92 | 10500 (1.10) | 13300 |
| B | 93 | 11000 (1.07) | 13400 |
| A^{a} | 93 | 7300 (1.11) | 13400 |
| B^{a} | 0 | - | - |

| | | | |
|---|---|---|---|
| *^{a} reaction performed with a batch* of *LA* *contaminated with 4 mol% of lactic acid.* | | | |

Conditions : Under argon, 288.03 mg (2.00 mmol) of rac-Lactide and 0.01 eq. of catalyst are weighed in a vial then mixed in the chosen solvent. Afterwards, 0.01 eq. of co-initiator (*ⁱ*PrOH and/or lactic acid) is added with a 5µL Hamilton then the vial is crimped and let stirred for 2h at the desired temperature.

## Claims

1. A compound of formula (I) : 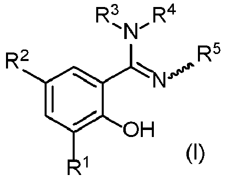 and stereoisomeric forms, mixtures of stereoisomeric forms,
and salts thereof,
Wherein :
R¹ and R² at each occurrence are independently selected from H, F, Cl, Br, I, C₁-C₆ alkyl, C₃-C₁₀ cycloalkyl, C₆-C₁₀ aryl or SiR⁶R⁷R⁸, said alkyl, cycloalkyl and aryl being optionally substituted by 1 to 3 R¹⁹ ;
R³ and R⁴ at each occurrence are independently selected from C₁-C₆ alkyl, C₃-C₁₀ cycloalkyl or C₆-C₁₀ aryl; or
R³ and R⁴ may form together with the nitrogen to which they are attached, a 3-7 membered heterocycloalkyl group,
said alkyl, cycloalkyl, aryl and heterocycloalkyl being optionally substituted by 1 to 3 R²⁰;
R⁵ is C₁-C₆ alkyl, C₁-C₆ perfluoroalkyl, C₃-C₁₀ cycloalkyl, C₆-C₁₀ aryl, C₆-C₁₀ perfluoroaryl, 3-7 membered heterocycloalkyl, 5-7 membered heteroaryl; said alkyl, cycloalkyl, aryl, heteroaryl and heterocycloalkyl being optionally substituted by 1 to 3 R²¹; or
R⁵ is -C(R⁹R¹⁰)-C(R¹¹R¹²)-Y, -Ar-Y, -Ar -Ar-Y or a group of formula (II) : 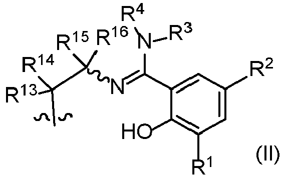
R⁹, R¹⁰, R¹¹and R¹² at each occurrence are independently selected from H, C₁-C₆ alkyl, C₃-C₁₀ cycloalkyl, C₆-C₁₀ aryl, said alkyl, cycloalkyl and aryl being optionally substituted by 1 to 3 R²²; or
R⁹ and R¹¹ represent H and R¹⁰ and R¹² form together with the carbon atoms to which they are attached a C₃-C₁₀ cycloalkyl group, said cycloalkyl being optionally substituted by 1 to 3 R²²; or
Ar is C₆-C₁₀ aryl group optionally substituted by 1 to 3 R²³;
R¹³, R¹⁴, R¹⁵ and R¹⁶ at each occurrence are independently selected from H, C₁-C₆ alkyl, C₃-C₁₀ cycloalkyl or C₆-C₁₀ aryl, said alkyl, cycloalkyl and aryl being optionally substituted by 1 to 3 R²⁴; or
R¹³ and R¹⁵ are H and R¹⁴ and R¹⁶ form together a C₃-C₁₀ cycloalkyl group optionally substituted by 1 to 3 R²⁴ ; or
R¹³, R¹⁴, R¹⁵ and R¹⁶ are combined to form together a C₆-C₁₀ aryl group optionally substituted by 1 to 3 R²⁴ ;
Y represent OH, C₁-C₆ alkoxy, NR¹⁷R¹⁸,
R¹⁷ and R¹⁸ at each occurrence are independently selected from H, C₁-C₆ alkyl, C₃-C₁₀ cycloalkyl or a C₆-C₁₀ aryl, said alkyl, cycloalkyl and aryl being optionally substituted by 1 to 3 R²⁵,
R⁶, R⁷ and R⁸ at each occurrence are independently selected from C₁-C₆ alkyl, or C₆-C₁₀ aryl, said alkyl and aryl being optionally substituted by 1 to 3 R²⁶,
R¹⁹, R²⁰, R²¹, R²², R²³, R²⁴, R²⁵ and R²⁶ at each occurrence are independently selected from C₁-C₄ alkyl, C₁-C₄ alkoxy or C₆-C₁₀ aryl,
With the proviso that :
- if R¹ and R² are both H, and R³ and R⁴ are both ethyl, then at least one of R¹³, R¹⁴, R¹⁵ and R¹⁶ is different from H,
- if R¹ and R² are both H, and R³ and R⁴ are both ethyl, then R⁵ cannot be propyl,
- if R¹ and R² are both H, and R³ and R⁴ are both methyl, then R¹³, R¹⁴, R¹⁵ and R¹⁶ cannot form together with the carbon atoms to which they are attached, a cyclohexyl group.

2. The compound of formula (I) of claim 1, which is a stereoisomeric form having a *trans* configuration :

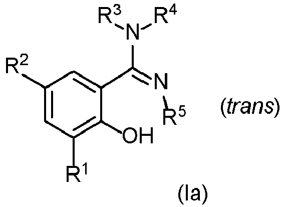

3. The compound of formula (I) of any of claims 1 or 2, wherein R⁵ is a group of formula (II) :

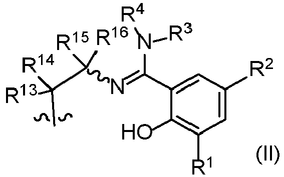

4. The compound of formula (I) of any of claims 1 to 3, which is an alcoholate salt of formula (Ib) :

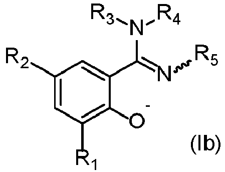

5. The compound of formula (I) of any of claims 1 to 4, which is a compound of formula (Ic) :

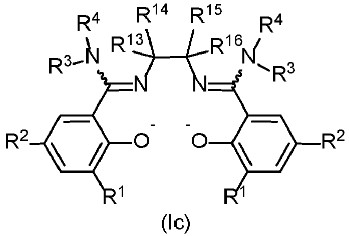

6. The compound of formula (I) of any of claims 1 to 5, wherein R¹ and R² at each occurrence are independently selected from H, or C₁-C₆ alkyl, preferably H, methyl, ethyl, isopropyl, t-butyl.

7. The compound of formula (I) of any of claims 1 to 6, wherein R³ and R⁴ at each occurrence are independently selected from C₁-C₆ alkyl, or C₃-C₁₀ cycloalkyl, notably methyl, ethyl, isopropyl, cyclohexyl, or R³ and R⁴ may form together with the nitrogen to which they are attached, a pyrrolidinyl or a piperidinyl group.

8. The compound of formula (I) of any of claims 1 to 7, wherein R⁵ is C₁-C₆ alkyl, C₃-C₁₀ cycloalkyl, C₆-C₁₀ aryl, C₆-C₁₀ perfluoroaryl, notably n-propyl, cyclohexyl, or C₆F₅.

9. The compound of formula (I) of any of claims 1 to 7, wherein Y is O⁻, OMe, N(Me)₂ or N(iPr)₂.

10. A coordination complex of formula (III) :
ML'mXn (III)
Wherein :
- M is a metal or a metalloid element of valence p ;
- X is an halogen atom, C₁-C₆ alkoxy group, N(R²⁷)₂ or C₁-C₆ alkyl;
- R²⁷ is a (C₁-C₆) alkyl group; a SiR¹⁶R¹⁷R¹⁸ group wherein R¹⁶, R¹⁷ and R¹⁸ are as defined in formula (I);
- m is 1, 2, 3 or 4 ;
- n is 0, 1, 2, or 3, provided that m+n=p,
- L' is a ligand of formula (I'b), 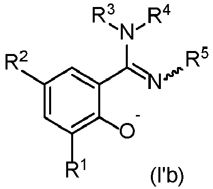
- wherein R¹, R², R³, R⁴ and R⁵ are as defined in any of claims 4 to 9, With the proviso that
- if R¹ and R² are both H, and R³ and R⁴are both methyl, then R¹³, R¹⁴, R¹⁵ and R¹⁶ cannot form together with the carbon atoms to which they are attached, a cyclohexyl group.

11. The coordination complex of claim 10, which is selected from (IIIa), (IIIb) or (IIIc) :

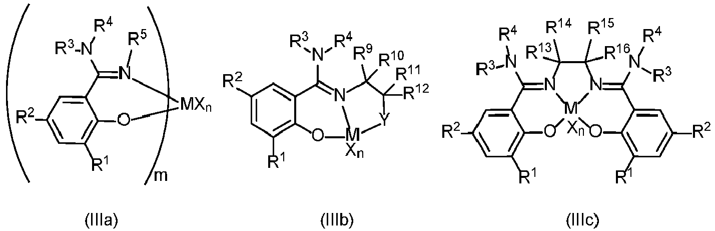

12. The coordination complex of any of claims 10 or 11, wherein M is selected from Ti, Zr, Zn, Al or B.

13. A use of a coordination complex of formula (IV) for catalyzing a ring opening polymerisation (ROP) of cyclic esters :
ML"mXn (IV)
Wherein
M, m and n are as defined in any of claims 10 to 12, and
L" is a ligand of formula (I"b), 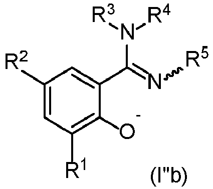 wherein R¹, R², R³, R⁴ and R⁵ are as defined in any of claims 4 to 9.

14. A catalyst for use in ring opening polymerisation (ROP) of cyclic esters comprising a coordination complex of formula (III), as defined in any of claims 10 to 12.

15. The catalyst of claim 14 which is the complex (IIIa₁) :
